(19) Europäisches Patentamt
European Patent Office
Office européen des brevets

(11) Publication number: **0 080 285**
**A1**

(12) # EUROPEAN PATENT APPLICATION

(21) Application number: **82305885.4**

(22) Date of filing: **05.11.82**

(51) Int. Cl.³: **C 07 D 498/04**, A 61 K 31/42
//
(C07D498/04, 263/00, 205/00),
(A61K31/42, 31/43)

(30) Priority: **25.11.81 GB 8135463**

(43) Date of publication of application: **01.06.83**
**Bulletin 83/22**

(84) Designated Contracting States: **CH DE FR GB IT LI NL**

(71) Applicant: **BEECHAM GROUP PLC, Beecham House Great West Road, Brentford Middlesex (GB)**

(72) Inventor: **Luk, Kong, 4 Palmer Close, Horley Surrey (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Therapeutic compounds containing beta-lactams.**

(57) A compound of formula (I) or a salt or ester thereof:

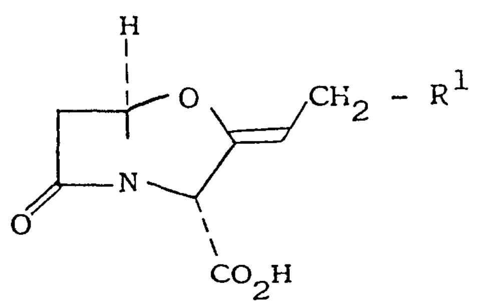

(I)

wherein $R^1$ is a $C_{2-6}$ alkyl, $C_{1-6}$ alkyl substituted by up to three fluorine atoms, $C_{2-6}$ alkenyl, $C_{2-6}$ alkynyl group or an aromatic hydrocarbon optionally substituted by up to three $C_{1-6}$ alkyl groups.

EP 0 080 285 A1

RJB/B.1154

0080285

# THERAPEUTIC COMPOUNDS CONTAINING β-LACTAMS

This invention relates to a class of novel β-lactam compounds, to the process for their preparation and to pharmaceutical compositions containing them.

U.K. Patent Nos. 1,508,977 and 1,508,978 disclose *inter alia* clavulanic acid and its salts and esters. Clavulanic acid has the formula (A):

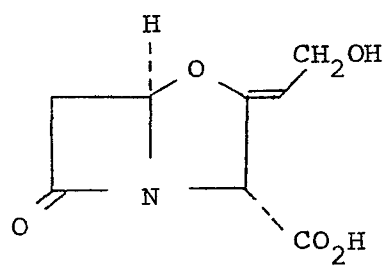

(A)

We have now found a novel class of compounds containing the clavam nucleus, which have anti-bacterial and β-lactamase inhibitory activity.

Accordingly, the present invention provides a compound of formula (I) or a salt or ester thereof:

(I) 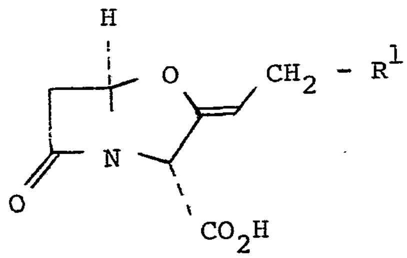

wherein $R^1$ is a $C_{2-6}$ alkyl, $C_{1-6}$ alkyl substituted by up to three fluorine atoms, $C_{2-6}$ alkenyl, $C_{2-6}$ alkynyl group or an aromatic hydrocarbon optionally substituted by up to three $C_{1-6}$ alkyl groups.

The major utility of the compound of formula (I) is as a pharmaceutical and, accordingly, the salts and esters of the compound of formula (I) are preferably pharmaceutically acceptable. The compound of formula (I) may also be used as an antibacterial or β-lactamase inhibitor in non-pharmaceutical uses such as, for example, as a disinfectant or paint additive; those salts and esters which are not normally considered to be pharmaceutically acceptable are suitable for this application.

Suitablealkyl groups for $R^1$ include the $C_{3-6}$ alkyl groups.

Suitable aromatic hydrocarbon for $R^1$ include phenyl and naphthyl optionally substituted by up to three $C_{1-6}$ alkyl groups.

Most suitably $R^1$ is n-propyl, n-butyl group or a phenyl group.

Suitable pharmaceutically acceptable salts of the compounds of formula (I) include metals salts, eg aluminium, alkali metal salts such as sodium or potassium, alkaline earth metal salts such as calcium or magnesium and ammonium or substituted ammonium salts, for example those with lower alkylamines such as triethylamine, cycloalkylamines such as bicyclohexylamine,

BAD ORIGINAL

or with dibenzylamine, N,N-dibenzylethylenediamine, 1-ephenamine, N-ethyl-piperidine, or N-benzyl-β-phenethylamine.

Examples of suitable pharmaceutically acceptable groups include those which break down readily in the human body to leave the parent acid or its salt, for example acyloxyalkyl groups such as acetoxymethyl, pivaloyloxymethyl, α-acetoxyethyl and α-pivaloyloxymethyl groups; alkoxycarbonyloxyalkyl groups, such as ethoxycarbonyloxymethyl; dialkylamino alkyl groups such as dimethylaminomethyl, dimethylaminoethyl, diethylaminomethyl or diethylaminoethyl; and α-ethoxycarbonyloxyethyl; and lactone groups such as phthalidyl or dimethoxyphthalidyl.

The present invention also provides a pharmaceutical composition which comprises a compound of formula (I) or a pharmaceutically acceptable salt or ester thereof and a pharmaceutically acceptable carrier.

The compositions of the invention include those in a form adapted for oral, topical or parenteral use and may be used for the treatment of the infection in animals including humans.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrant and the like in accordance with conventional pharmaceutical practice in the manner well understood by those skilled in the art of formulating anti-biotics.

Injectable or infusable compositions of a compound of the invention are particularly suitable as high blood levels of the compound can occur after administration by injection or infusion. Thus, one preferred composition aspect of this invention comprises a compound of the invention in sterile form and most suitably in sterile crystalline form.

BAD ORIGINAL

The injectable solution of the compound of this invention may be made up in a sterile pyrogen-free liquid such as water, aqueous ethanol or the like.

An alternative approach to administering the compounds of this invention is to utilise an injectable suspension. Such suspensions may be made up in sterile water; sterile saline or the like, and may also contain suspending agents such as polyvinylpyrrolidone, lecithin or the like. Alternatively such compositions may be prepared in an acceptable oil suspending agent such as arachis oil or its equivalent. For use in such suspensions the compounds of this invention should be in the form of fine particles.

Unit dose compositions comprising a compound of this invention adapted for oral administration form a further suitable composition aspect of this invention.

Unit dose compositions comprising a compound of this invention adapted for topical administration are also presented by this invention. In this instance 'topical administration' also includes local administration to internal surfaces of mammary glands of cattle, for example during the treatment of mastitis by intra-mammary administration.

The compound of the formula may be present in the composition as sole therapeutic agent or it may be present together with other therapeutic agents such as a penicillin or cephalosporin. Considerable advantages accrue from the inclusion of a penicillin or cephalosporin which shows instability to β-lactamases since the resulting composition shows enhanced effectiveness (synergy). Suitable penicillins, cephalosporins or other β-lactam anti-biotics for inclusion in such synergistic compositions include not only those

BAD ORIGINAL

known to be highly susceptible to β-lactamases but also those which have a degree of intrinsic resistance to β-lactamases.

Suitable penicillins for inclusion in the compositions of this invention include benzylpenicillin, phenoxymethylpenicillin, carbenicillin, azidocillin, propicillin, ampicillin, amoxycillin, epicillin, ticarcillin, cyclacillin, pirbenicillin, azlocillin, mezlocillin, sulbenicillin, pipericallin, and other known penicillins including pro-drugs therefor such as their in vivo hydrolysable esters such as the acetoxymethyl, pivaloyloxymethyl, α-ethoxycarbonyloxyethyl or phthalidyl esters of ampicillin, benzylpenicillin or amoxycillin, and aldehyde or ketone adducts of penicillins containing a 6-α-aminoacetamide side chain (such as hetacillin, metampicillin and analogous derivatives of amoxycillin) or α-esters of carbenicillin or ticarcillin such as their phenyl or indanyl α-esters.

Suitable cephalosporins for inclusion in the compositions of this invention include cefatrizine, cephaloridine, cephalothin, cefazolin, cephalexin, cephacetrile, cephamandole nafate, cephapirin, cephradine, 4-hydroxycephalexin, cefaparole, cephaloglycin, cefoperazone and other known cephalosporins or pro-drugs thereof.

Such compounds are frequently used in the form of a salt or hydrate of the like.

Naturally if the penicillin or cephalosporins in the composition is not suitable for oral administration then the composition will be adapted for parenteral administration.

Highly favoured penicillins for use in the compositions of this invention include ampicillin, amoxycillin, carbenicillin and ticarcillin. Such penicillins may be used as a pharmaceutically acceptable salt such as the sodium salt. Alternatively the ampicillin or amoxycillin may be used in the form of fine particles of the zwitterionic form (generally as ampicillin trihydrate or amoxycillin trihydrate) for use in an injectable suspension, for example in the manner hereinbefore described for a compound of this invention.

The preferred penicillin for use in the synergistic composition is amoxycillin, for example as its sodium salt or trihydrate.

Particularly suitable cephalosporins for use in the compositions of this invention include cephaloridine and cefazolin which may be in the form of a pharmaceutically acceptable salt for example the sodium salt.

When present together with a cephalosporin or penicillin, the ratio of a compound of the invention to the penicillin or cephalosporin agent may vary over a wide range of ratios such as from 10:1 to 1:10 for example about 3:1, 2:1, 1:1, 1:2, 1:3, 1:4, 1:5 or 1:6, (wt/wt, based on pure free anti-biotic equivalent). Orally administrable compositions containing a compound of the invention will normally contain relatively more synergist than corresponding injectable compositions.

The total quantity of a compound of the invention in any unit dosage form will normally be between 25 and 1000 mg and will usually be between 50 and 500 mg, for example about 62.5, 100, 125, 150, 200 or 250 mg.

BAD ORIGINAL

Compositions of this invention may be used for the treatment of infections of inter alia the respiratory tract, the urinary tract and soft tissues in humans and mastitis in cattle.

Normally between 50 and 3000 mg of the compounds of the invention will be administered each day of treatemnt but more usually between 100 and 1000 mg of the compounds of the invention will be administered per day, for example at 1-6 doses, more usually as 2, 3 or 4 doses. However, for the treatment of more severe systemic infections or infections of particularly intransigent organisms higher doses may be used in accordance with clinical practice.

The penicillin or cephalosporin in the synergistic composition of this invention will normally be present at approximately the amount at which it is conventionally used which will usually be expected to be from about 62.5 to 3000 mg per dose, more usually about 125, 250, 500 or 1000 mg per dose.

One particularly favoured composition of this invention will contain from 150 to 1000 mg of amoxycillin as the trihydrate or sodium salt and from 25 to 500 mg of a compound of this invention.

A further particularly favoured composition of this invention will contain from 150 to 1000 mg of ampicillin or a pro-drug therefor and from 25 to 500 mg of a compound of this invention.

Most suitably this form of composition will contain ampicillin trihydrate, ampicillin anhydrate, sodium ampicillin, hetacillin, pivampicillin hydrochloride,

bacampicillin hydrochloride, or talampicillin hydrochloride. Most suitably this form of the composition will contain a compound of the formula (I) when in crystalline form.

Most suitably the preceding composition will contain from 200 to 700 mg of the penicillin component. Most suitably the preceding composition will comprise from 50 to 250 mg of a compound of the formula (I) preferably in crystalline form.

Such compositions may be adapted for oral or parenteral use except when containing an in vivo hydrolysable ester of ampicillin or amoxycillin in which case the compositions will not be adapted for parenteral administration.

Another particularly favoured composition of this invention will contain from 200 to 2000 mg of carbenicillin, ticarcillin or a pro-drug therefor and from 50 to 500 mg of a compound of the invention.

Suitably this form of composition will contain di-sodium carbenicillin. Suitably this form of the composition will contain di-sodium ticarcillin.

More suitably this form of the composition will contain from 75 to 250 mg of a compound of the formula (I) preferably in crystalline form. Such compositions containing di-salts of carbenicillin and ticarcillin will be adapted for parenteral administration.

The present invention also provides a method of treating bacterial infections in humans or domestic mammals which comprises the administration of a compositions of this invention.

Commonly the infection treated will be due to a strain of Staphylococcus aureus, Klebsiella aerogenes, Escherichia coli, Proteus sp., Bacteroides fragilis or the like. The organisms believed to be most readily treated by an antibacterially effective amount of a compound of this invention is Staphylococcus aureus. The other organisms named are more readily treated by using a synergistically effective amount of the compound of the invention and a penicillin or cephalosporin. The administration of the two components may take place separately but in general we prefer to use a composition containing both the synergist and the penicillin or cephalosporin.

The indications for treatment include respiratory tract and urinary tract infections in humans and mastitis in cattle.

The present invention also provides a process for the preparation of a compound of formula (II) or a salt or ester thereof:

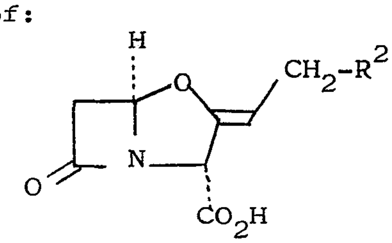

(II)

wherein $R^2$ is a group $R^1$ as defined with respect to formula (I) or a methyl group, which process comprises reacting a compound of formula (II):

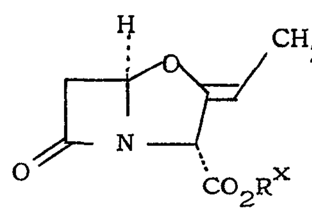

(III)

wherein $R^x$ is a carboxy protecting group, and $R^3$ is chlorine, bromine, iodine or a group $-OR^4$, wherein $R^4$ is

BAD ORIGINAL

an etherifying or esterifying group, with an organometallic base of formula $R^2Li$, $R^2Cu$ or a Grignard reagent of formula $R^2MgX$ wherein X is chlorine, bromine or iodine; and thereafter where necessary carrying out one or more of the following steps:

(a) removing the carboxy protecting group $R^x$; or

(b) converting a salt to the free carboxylic acid or to an ester, or to a different salt.

Compounds of formula (III) wherein $R^3$ is chlorine, bromine or iodine may be prepared by the methods described in German OLS 2657081.

Compounds of formula (III) wherein $R^3$ is a group $-OR^4$ may be prepared by the methods disclosed in U.K. Patent No. 1565209.

The present process when performed using the organolithium or Grignard reagent is suitably performed in the presence of cuprous ions. A preferred reagent for use in the present process is the organocopper lithium reagent of formula $LiCuR^2_2$.

Suitable groups $R^3$ in the compound of formula (III) include group $-OR^4$ wherein $R^4$ is a $C_{1-6}$ alkyl group optionally substituted by a $C_{1-6}$ alkoxy group. Preferably $R^4$ is a methoxymethyl, ethoxymethyl group or dichloroacetyl group.

Suitable carboxy protecting groups for the group $-CO_2R^x$ in formula (III) include ester derivatives of the carboxylic acid. The derivative is preferably one which may readily be cleaved at a later stage of the reaction.

Suitable ester-forming carboxyl-blocking groups are those which may be removed under conventional conditions. Such groups for $R^x$ include benzyl, p-methoxybenzyl,2,4,6-trimethylbenzyl, 3,5-di-t-butyl-benzyl, 4-pyridylmethyl, allyl, diphenylmethyl, triphenylmethyl, 2-benzyloxyphenyl, 4-methylthiopropyl, methoxymethyl, a silyl, or phosphorus-V-containing group, or methyl or ethyl.

The carboxylic group of a salt thereof may be regenerated from any of the above esters by usual methods appropriate to the particular $R^x$ group, for example, base-catalysed hydrolysis, or by enzymically-catalysed hydrolysis, or by hydrogenation.

The preceding reaction normally takes place in a solvent inert under the reaction conditions such as diethyl ether, di-isopropyl ether, tetrahydrofuran or dioxane. The reaction is generally carried out at a depressed or non-elevated temperature, for example, $-80^oC$ to $+30^oC$, and preferably at a depressed temperature, for example $-80^o$ to $0^oC$, and conveniently at about $-70^oC$.

Acids within formula (I) may also be prepared by the careful acidification of a corresponding salt such as the sodium salt.

Salts within formula (I) may be prepared by treatment of an acid within formula (I) with, for example, sodium ethyl hexanoate or potassium ethyl hexanoate. Salts within formula (I) may also be prepared by salt exchange in conventional manner, for example a solution of the lithium salt in water may be passed through a bed of ion exchange resin in the sodium form (eg Amberlite 120; a sodium salt of a sulphonated polystyrene divinyl benzene

co-polymer) in about ten-fold excess until elution is complete; the resulting sodium salt may be obtained by freeze-drying or by evaporation to crystallisation. Similarly, a sodium salt may be converted to a lithium salt or to a potassium salt in a similar manner.

The following Examples illustrate the invention.

Example 1

Ethoxymethyl 9-methyl-9-deoxyclavulanate

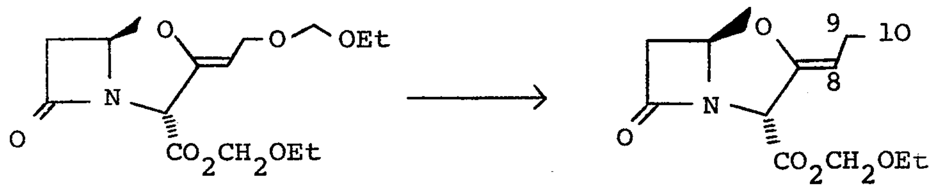

Methyllithium (2M solution in ethyl, 31 ml) was added to a stirred suspension of cuprous iodide (5.9 g) in dry ether (30 ml) at $0^o$ under nitrogen. The mixture was maintained at $0^o$ for 0.5 hr and then cooled to $-70^o$. Ethoxymethyl 9-O-ethoxymethylclavulanate (2.0 g) in ether (10 ml) was then added and the solution was stirred at $-70^o$ for 1 hr. A mixture of ethyl acetate (80 ml) and aqueous saturated ammonium chloride solution (100 ml) was added, which was then allowed to come to room temperature. The organic layer was separated and washed with dilute hdyrochloric acid, saturated aqueous sodium bicarbonate, brine, dried, and evaporated. The residue was chromatographed over silica gel (20 g). Elution of the column with petrol-ethyl acetate (2:1) gave the title compound (0.3 g, 19%), $[\alpha]_D^{20}$ + 48.7° (c, 1.3; $CHCl_3$), $\nu_{max}$ ($CHCl_3$) 1800, 1750, 1695, 1300 and 1120 $cm^{-1}$, $\delta_H$ ($CDCl_3$) 0.95 (3H, t, J = 8Hz, 10-$CH_3$), 1.20 (3H, t, J = 8Hz, $OCH_2CH_3$), 2.10 (2H, quintet, J = 8Hz, 9-$CH_2$), 3.00 (1H, d, J = 17Hz, 6β-CH), 3.45 (1H, dd, J = 2.5Hz, $J^1$ = 17Hz, 6α-CH), 3.65 (2H, q, J = 8Hz, $OCH_2CH_3$), 4.60 (1H, t, J = 8Hz, 8-CH), 5.00 (1H, s, 3-CH), 5.30 (2H, ABq, J = 7Hz, $-OCH_2O-$), and 5.65 (1H, d, J = 2.5Hz, 5-CH).
(Found $M^+$ 255.1105. $C_{12}H_{17}NO_5$ requires $M^+$ 255.1107).

Example 2

Sodium 9-methyl-9-deoxyclavulanate

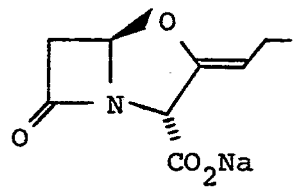

Lithium bromide (0.3 g) was added to a solution of ethoxymethyl 9-methyl-9-deoxyclavulanate (0.1 g) in N,N-dimethylformamide (6 ml) and the mixture was stirred at room temperature overnight. The reaction mixture was then filtered and the filtrate concentrated to 1 ml. This was diluted with ethyl acetate (10 ml) and washed with brine (2 x 10 ml). Water (15 ml) was then added and the pH adjusted to 7.5 with aqueous sodium hydroxide (0.1 M). The aqueous layer was separated and freeze-dried to give the title compound (0.05 g) as a white solid, $[\alpha]_D^{20}$ + 42.6° (c, 1.1; $H_2O$), $\nu_{max}$ (KBr) 1805, 1780, 1760 and 1610 $cm^{-1}$, $\delta_H$ (DMSO-$d_6$) 0.90 (3H, t, J = 8Hz, 10-$CH_3$), 2.00 (2H, quintet, J = 8Hz, 9-$CH_2$), 2.90 (1H, d, J = 17Hz, 6β-CH), 3.40 (1H, d, J = 2.5Hz, J = 17Hz, 6α-CH), 4.40 (1H, s, 3-CH), 4.40 (1H, t, J = 8Hz, 8-CH), and 5.50 (1H, d, J = 2.5Hz, 5-CH).

Example 3

Benzyl 9-vinyl-9-deoxyclavulanate

Vinylmagnesium bromide in pentane (5ml, 1.3M) was added to a suspension of cuprous iodide (0.63g) in tetrahydrofuran (20ml) at -78$^o$ under nitrogen. The mixture was allowed to warm up to 0$^o$ over 15 minutes to give a fine brownish yellow suspension which was then cooled down to -78$^o$. A solution of benzyl 9-O-methoxymethlclavulanate (0.36g) in tetrahydrofuran (10ml) was then added and the mixture was stirred at -78$^o$ under nitrogen for 2 hours. It was then quenched with acetic acid (0.5ml) and allowed to come to room temperature. The mixture was then partitioned between brine and ethyl acetate. The organic extract was dried, evaporated to dryness and the residue was chromatographed over silica gel (12g). Elution of the column with petrol-ethyl acetate (4:1) gave the title compound, $[\alpha]_D^{20}$ + 32.9 (c, 1.0; $CHCl_3$), $\nu_{max}$ ($CHCl_3$) 1795, 1740, and 1635 $cm^{-1}$, $\delta_H$($CDCl_3$) 7.35 (5H, m, ArH), 5.75(1H, m, -CH=$CH_2$), 5.67 (1H,d,J = 3Hz, 5-CH), 5.20 (2H, ABq, J= 12Hz, -$CH_2$PH), 5.05 (1H, broad s, 3-CH), 4.90-5.02(2H, m,-CH =$CH_2$), 4.62 (1H, broad t,J=7Hz, 8-CH), 3.46 (1H, dd, J= 18Hz, $J^1$=3Hz, 6α-CH), 3.06 (1H, d, J=18Hz, 6β-CH), and 2.82 (2H, m, 9-CH).

0080285

Example 4

(2R,5R)Z-3-butylidene 2-benzyloxycarbonyl 7-oxo 4-oxa 1-azabicyclo[3.2.0]heptane

A solution of ethyllithium in ether (7 ml of a 1.5N solution)was added to a stirred suspension of cuprous iodide (0.95 gm) in dry ether (10 ml) cooled to -40°C under nitrogen. The rate of addition was such that the temperature did not rise above -30°C. When the addition was complete the mixture was maintained at -30°C for ½ hr and then cooled to -60°C. A solution of benzyl 9-0-dichloroacetylclavulanate (1 gm) in ether was then added and the mixture stirred at -60°C for 1 hr. The solution was then removed from the cooling bath and shaken well with ammonium chloride solution and filtered through Celite. The filtrate was extracted with ethyl acetate and the ethyl acetate solution was washed successively with water, sodium bicarbonate solution, water and brine. The solution was dried over magnesium sulphate and evaporated. The product was isolated by column chromatography of the residue using gradient elution (Kieselgel 5 : 1 going to 2 : 1 petroleum ether : ethyl acetate as eluent).

I.R. $\nu_{max.}$ ($CHCl_3$) 1800, 1750, 1700 $cm^{-1}$.

N.M.R. $\delta$ ($CDCl_3$) 0.84 (3H, t, J = 7 Hz), 1.1 - 1.55 (2H, apparent sextet, J = 7 Hz) 2.03 (2H, apparent q, J = 7 Hz), 2.97 (1H, d, J = 17 Hz), 3.41 (1H, dd, J = 3 and 17 Hz), 4.55 (1H, t, J = 7 Hz), 5.0 (1H, s), 5.17 (2H, s), 5.60 (1H, d, J = 3 Hz) 7.33 (5H, s).

0080285

Example 5

Lithium (2R,5R) Z-3-butylidene 7-oxo 4-oxa-1-áza-bicyclo [3.2.0]heptane 2-carboxylate

A solution of (2R,5R) Z-3-butylidene 2-benzyloxycarbonyl 7-oxo 4-oxa 1-azabicyclo [3.2.0.]heptane (0.177 gm) in tetrahydrofuran (5 ml) was hydrogenated over 10% palladium charcoal (50 mgs) for 20 minutes. The solution was filtered through Celite and the filter cake washed with tetrahydrofuran. The solution was evaporated to about 3 ml and treated with a solution of lithium carbonate (21.8 mgs) in water (5 ml). The remaining tetrahydrofuran was evaporated and the aqueous solution was washed twice with ethyl acetate and filtered through Celite. The solution was evaporated to very low volume and the residue triturated with acetone. The crystalline solid was filtered off and dried under vacuum over phosphorus pentoxide. Yield 81 mgs.

I.R. $\nu_{max.}$ (KBr) 1768, 1701, 1614 $cm^{-1}$.

N.M.R. $\delta$ ($D_2O$) 0.78 (3H, t, J = 7 Hz) 1.29 (2H, apparent sextet, J = 7 Hz) 1.97 (2H, apparent q, J = 7 Hz), 2.95 (1H, d, J = 7 Hz) 3.45 (1H, dd, J = 3 and 17 Hz), 4.64 (1H, t, J = 7 Hz), 5.59 (1H, d, J = 3 Hz).

Example 6

(2R,5R) Z-3-hexylidene 2-benzyloxycarbonyl 7-oxo-4-oxa 1-azabicyclo[3.2.0]heptane

A stirred suspension of cuprous iodide (0.95 gm) in dry ether (10 ml) was cooled in an ice bath and then a solution of butyllithium in cyclohexane (3.8 ml of 2.6N solution) was added. The mixture was allowed to warm to room temperature for ½ hr. The mixture was cooled in a dry-ice/acetone bath and a solution of benzyl 9-O-dichloroacetylclavulanate (0.8 gm) in ether (4 ml) was added in one portion. The solution was stirred at -78°C for 1½ hr and then removed from the cooling bath. The solution was treated with saturated ammonium chloride solution and the mixture extracted three times with ethyl acetate. The ethyl acetate solution was washed successively with water, sodium bicarbonate solution, water and brine. The solution was dried over magnesium sulphate and evaporated. The product was isolated by column chromatography of the residue using gradient elution (Kieselgel 4 : 1 going to 2 : 1 petroleum ether : ethyl acetate). Yield, 0.074 gm.

I.R. $\nu_{max.}$ ($CHCl_3$) 1805, 1750 and 1700 $cm^{-1}$. N.M.R. $\delta$ ($CDCl_3$) 0.85 (3H, t, J = 6 Hz), 1.1 - 1.4 (6H, m), 2.04 (2H, q, J = 6 Hz), 2.97 (1H, d, J = 17 Hz), 3.41 (1H, dd, J = 3 and 17 Hz), 4.54 (1H, broad t, J = 7 Hz), 5.00 (1H, s), 5.17 (2H, s), 5.61 (1H, d, J = 3 Hz) 7.32 (5H, s).

Example 7

Lithium (2R,5R) Z-3-hexylidene 7-oxo 4-oxa 1-azabicyclo-[3.2.0]heptane 2-carboxylate

A solution of (2R,5R) Z-3-hexylidene 2-benzyloxycarbonyl 7-oxo 4-oxa 1-azabicyclo [3.2.0]heptane (0.36 gm) in tetrahydrofuran (10 ml) was hydrogenated over 10% palladium charcoal (0.1 gm) for 15 minutes. The solution was filtered through Celite and the filter cake washed with tetrahydrofuran. The tetrahydrofuran solution was evaporated to about 5 ml and diluted with water (5 ml). Lithium carbonate (40 mgs) was added and when it had dissolved the remaining tetrahydrofuran was evaporated. The aqueous solution was washed twice with ethyl acetate and filtered through Celite. The solution was then evaporated almost to dryness and triturated with acetone. The solid was filtered off, washed with acetone and dried over phosphorus pentoxide under vacuum. Yield 0.176 mg.

I.R. $\nu_{max.}$ (KBr) 1765, 1697 and 1612 $cm^{-1}$.

N.M.R. $\delta$ ($D_2O$) 0.83 (3H, t, 6 Hz), 1.1 - 1.5 (6H, m), 1.9 - 2.2 (2H, m), 2.93 (1H, d, J = 17 Hz),3.51 (1H, dd, J = 3 and 17 Hz), 4.69 (1H, t, J = 7 Hz), 4.79 (1H, s), 5.63 (1H, d, J = 3 Hz).

Example 8

(2R,5R) Z-3-(2-phenyl)ethylidene 2-benzyloxycarbonyl 7-oxo 4-oxa 1-azabicyclo[3.2.0]heptane

A solution of phenyllithium in ether/cyclohexane (10.4 ml of 1.9M) was added to a suspension of cuprous iodide (1.9 gm) in dry ether (30 ml) stirred under nitrogen. The solution was stirred at room temperature for 40 minutes and then cooled to -78°C. A solution of benzyl 9-0-dichloroacetylclavulanate (2 gm) in ether (10 ml) was then added. The mixture was stirred at -78°C for 1hr and then vigorously shaken with ammonium chloride solution. The solution was filtered through Celite and the filtrate extracted twice with ethyl acetate. The ethyl acetate solution was washed successively with water, sodium bicarbonate solution, water and brine. The solution was dried over magnesium sulphate and evaporated. The product was isolated by column chromatography of the residue using gradient elution (Kieselgel 5 : 1 going to 3 : 1 petroleum ether : ethyl acetate). Yield 0.127 gm.

I.R. $\nu_{max.}$ ($CHCl_3$) 1800, 1750 and 1695 $cm^{-1}$. N.M.R. $\delta$ ($CDCl_3$) 3.00 (1H, d, J = 17 Hz), 3.40 (2H, d, J = 7 Hz), 3.44 (1H, dd, J = 3 and 17 Hz), 4.79 (1H, broad t, J = 7 Hz), 5.07 (1H, s) 5.15 (2H, s), 5.67 (1H, d, J = 3 Hz), 7.0 - 7.4 (10H, m).

BAD ORIGINAL

Example 9

Lithium (2R,5R) Z-3-(2-phenyl)ethylidene 7-oxo 4-oxa 1-azabicyclo[3.2.0]heptane-2-carboxylate

A solution of (2R,5R) Z-3-(2-phenyl)ethylidene 2-benzyloxycarbonyl 7-oxo 4-oxa 1-azabicyclo[3.2.0]-heptane (0.127 gm) in tetrahydrofuran (5 ml) was hydrogenated over 10% palladium charcoal (30 mgs) for 20 minutes. The solution was filtered through Celite and the filter cake washed with tetrahydrofuran. The solution was evaporated to about 2 ml and then diluted with water (2 ml). Lithium carbonate (13.4 mgs) was then added and when solution was complete the remaining tetrahydrofuran was evaporated. The aqueous solution was washed twice with ethyl acetate and filtered through Celite. The solution was evaporated almost to dryness and the residue triturated with acetone/ ether. The crystalline solid was filtered off and dried under vacuum over phosphorus pentoxide. Yield 48 mgs.

I.R. $\nu_{max.}$ (KBr) 1765, 1697, 1610 $cm^{-1}$. N.M.R. $\delta$ ($D_2O$) 3.02 (1H, d, J = 17 Hz), 3.3 - 3.65 (3H, m), 4.89 (1H, s), 4.92 (1H, t, J = 7 Hz), 5.69 (1H, d, J = 3 Hz), 7.29 (5H, s).

CLAIMS

1. A compound of formula (I) or a salt or ester thereof:

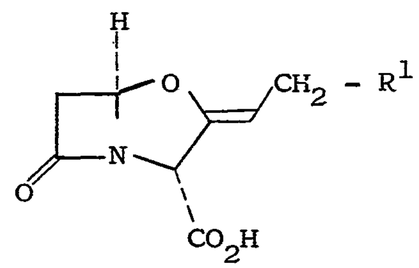

(I)

wherein $R^1$ is a $C_{2-6}$ alkyl, $C_{1-6}$ alkyl substituted by up to three fluorine atoms, $C_{2-6}$ alkenyl, $C_{2-6}$ alkynyl group or an aromatic hydrocarbon optionally substituted by up to three $C_{1-6}$ alkyl groups.

2. A compound as claimed in claim 1 wherein the salt or ester is pharmaceutically acceptable.

3. A compound as claimed in claim 1 or claim 2 wherein $R^1$ is $C_{3-6}$ alkyl.

4. A compound as claimed in claim 1 or claim 2 wherein $R^1$ is phenyl or naphthyl optionally substituted by up to three $C_{1-6}$ alkyl groups.

5. A compound as claimed in claim 1 or claim 2 wherein $R^1$ is n-propyl, n-butyl or phenyl.

6. A pharmaceutical composition comprising a compound as claimed in claim 2 and a pharmaceutically acceptable carrier.

7. A pharmaceutical composition as claimed in claim 6 which further comprises a penicillin.

8. A process for the preparation of a compound of formula (II) or a salt or ester thereof:

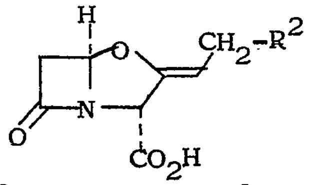

(II)

wherein $R^2$ is a group $R^1$ as defined with respect to formula (I) or a methyl group, which process comprises reacting a compound of formula (III):

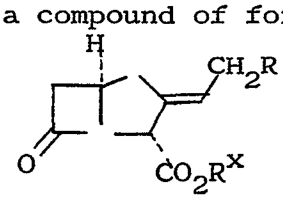

(III)

wherein $R^x$ is a carboxy protecting group, and $R^3$ is chlorine, bromine, iodine or a group $-OR^4$, wherein $R^4$ is an etherifying or esterifying group, with an organometallic base of formula $R^2Li$, $R^2Cu$ or a Grignard reagent of formula $R^2MgX$ wherein X is chlorine, bromine or iodine; and thereafter where necessary carrying out one or more of the following steps:

(a) removing the carboxy protecting group $R^x$; or

(b) converting a salt to the free carboxylic acid or to an ester, or to a different salt.

9. A process as claimed in claim 8 wherein the organometallic base is an organo copper lithium reagent of formula $LiCuR^2_2$

10. A compound as claimed in claim 1 for use as an antibacterial agent

European Patent Office

# EUROPEAN SEARCH REPORT

Application number

EP 82 30 5885

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 024 372 (BEECHAM) *Claims* | 1,6-8 | C 07 D 498/04<br>A 61 K 31/42 //<br>(C 07 D 498/04<br>C 07 D 263/00<br>C 07 D 205/00 )<br>(A 61 K 31/42<br>A 61 K 31/43 ) |
| Y | GB-A-1 593 275 (GLAXO) *Claims* | 1,6-8 | |
| A | EP-A-0 018 305 (HOECHST) *Claims 30-59* | 1,6,7 | |
| A | US-A-4 293 555 (B.G.CHRISTENSEN) *Column 1, lines 16-45* | 1,6 | |
| | ----- | | TECHNICAL FIELDS SEARCHED (Int. Cl. 3) |
| | | | C 07 D 498/00<br>A 61 K 31/00 |

The present search report has been drawn up for all claims

| Place of search | Date of completion of the search | Examiner |
|---|---|---|
| THE HAGUE | 14-02-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82